# EUROPEAN PATENT APPLICATION

(11) **EP 1 236 517 A1**
(43) Date of publication of application: **04.09.2002**
(21) Application number: 01103653.0
(22) Date of filing: 23.02.2001
(51) Int. Cl.: B05B 17/06, A61M 15/00

(54) **Method of manufacturing a liquid droplet spray device and such spray device**

(71) Applicant: Microflow Engineering SA, 2007 Neuchâtel (CH)
(72) Inventor: Hess, Joseph, 2022 Bevaix (CH); Luginbuhl, Philippe, 2518 Nods (CH); Flick, Jean-Marc, 2065 Savagnier (CH); Weber, Raphael, 2300 La Chaux-de-Fonds (CH); Hu, Bo, 2034 Peseux (CH)
(74) Representative: Balsters, Robert

(57) **Abstract**

The invention concerns a liquid droplet spray device for atomising a liquid substance. Its housing consists of at least a first substrate (15), a space (12) within the housing for containing the supplied liquid substance, an outlet means arranged in the first substrate (15) and comprising at least one outlet nozzle (19) and at least one output channel (20), said output channel (20) having straight side-walls, and a vibrating element (18) for vibrating the liquid so as to eject it.

According to the present invention, each output channel (20) has a stepped shape having a lower portion (20a) and an upper portion (20b), the lower portion being arranged adjacent the space (12) and being of larger diameter than the upper portion (20b).

The invention also concerns a method of manufacturing such device using a differential etching technique to obtain the stepped shape output channel.

## Description

The present invention relates to a method of manufacturing a liquid droplet spray device suitable for atomising a liquid substance such as a drug, a fragrance or other aerosolised liquids, as well as the device thus obtained. Such a device may be used, e.g., for perfume dispensers or for administrating a nebulized drug to a patient by means of his or her respiratory system. Such an administration device, in its simplest form, is commonly called an inhaler. It may be used, e.g., for the controlled administration of drugs or for a variety of therapies using nebulized drug administration including anaesthetics or during minimally invasive surgery. The device delivers the drug, which is in the form of a liquid substance, as a dispersion of atomised droplets. More specifically, the present invention concerns an improved liquid droplet spray device which efficiently creates and which fully expels a liquid droplet spray, as well as a method of manufacturing such.

Various devices are known for atomising a liquid. Document EP 0 516 565 describes an ultrasonic wave nebuliser which atomises water. This apparatus is used as a room humidifier. Vibration is transmitted through the water to the water surface from which the spray is produced. A perforate membrane is provided to retain the water in absence of oscillation.

Typically, inhaler devices use the same principle to atomise the liquid into droplets, see for example the document WO 95/15822.

Furthermore, the droplet size, which depends on the size of the outlet orifices of the perforate membrane, also depends on the vibration frequency. In order to obtain a small droplet, a very high frequency should be used, typically over 1 MHz for droplets of about 10 µm in diameter. This leads to increased power consumption due to the high frequency so that such a device is not suitable for a small battery operated device.

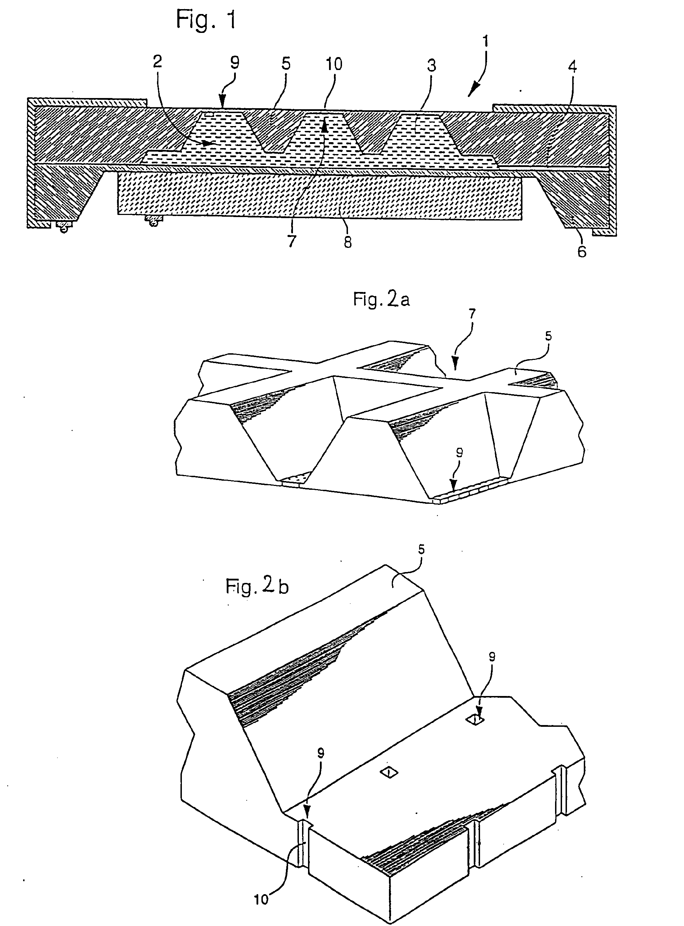

Another liquid droplet spray device is known from the document EP-A-0 923 957 and the document EP-A-1 005 916, both in the name of the present Applicant. A brief description of the liquid droplet spray device known from these documents, which are hereby incorporated by reference, is given here while referring to Figure 1.

In this particular embodiment spray device 1 consists of a housing formed of a superposition of a first, or a top substrate 5 and a second, or a bottom substrate 6 in-between which a chamber or a space 2 is formed for containing a liquid substance 3 and thus providing a compression chamber. Top substrate 5 contains outlet means consisting of cavity or cavities 7 which can partly constitute space 2, outlet nozzles 9 and output channels 10 connecting these nozzles to the cavity or cavities 7.

Liquid substance 3 enters spray device 1 by way of, e.g., a very low pressure, e.g., around a few millibar, or capillary action. This can be achieved for example by way of at least one input tube or needle 4 through which the liquid substance may be supplied from an external reservoir (not shown) into spray device 1. Spray device 1 further comprises a vibrating element 8, e.g. a piezoelectric element to cause vibration of liquid substance 3 in space 2.

The method of manufacturing this device is carried out by using technology known from the field of semiconductors. Thus, top and bottom substrates may be manufactured in a similar manner e.g. by etching a silicon wafer in a suitable manner, e.g. by wet or dry etching and by using one or more masks or by micro-machining Pyrex wafers. The substrates 5 and 6 are attached to each other, preferably by appropriate bonding technique, such as anodic bonding, so as to form and enclose space 2.

The method of manufacturing the output channel preferably comprises micro-machining the channel by using a deep reactive vertical plasma etching of silicon, e.g. at room temperature or low temperature, and an advanced silicon etch process so as to obtain highly-toleranced straight side-walls of the channel. These prior art documents further describe techniques allowing for such output channels with a straight, non-tapered profile. This provides for a precisely defined pressure drop, droplet size and flow behaviour across output channel 10 for aqueous solutions and suspensions whereas the relatively smooth surface is suited for medications carrying small solid particles, e.g. from less than 1 to approx 2 µm, in suspensions. The same effect can be obtained proportionally with larger dimensions, e.g. with nozzles of 10 µm or larger for example for perfume dispensing applications or in a practical variation of the cited prior art of the applicant by simply using the vertical plasma etching micro-machining method to produce an output channel whose cross-section is divided into two or more identical sub-channels to allow for an even finer control of pressure drop, droplet size and flow behaviour across said channel 10. The cross section of the vertical channel or channel section can be of a suitable geometrical form, e.g. circular, triangular or a suitable geometrical shape such as a cross when the channel consists of several identical sub-channels. The cross section of the cavities 7 can also be of suitable geometrical form or combination of forms.

Figure 2a shows a schematic detailed view of the first, or top substrate of this prior art liquid droplet spray device. The top substrate is shown upside down with respect to Figure 1 in a further practical variation of the cited prior art which has already been shown with an inversion of the bottom substrate, thus further reducing dead space. As can be seen, top substrate 5 comprises the cavities 7, output channels 10 and outlet nozzles 9. The top surface of the substrate-delimiting cavity 7 forms a membrane section in substrate 5. The surface of this membrane section is much larger than the actual nozzle surface, so that it is possible to provide several outlet nozzles 9 on the membrane surface in order to eject more droplets simultaneously. As already mentioned in the cited prior art, it is obvious that cavities 7 are not necessarily tapered but can be straight according to the manufacturing process chosen. Figure 2b shows a close-up view of a part of figure 2a in which it can be seen that the output channels 10 and outlet nozzles 9 may be readily placed according to the specific conditions.

The diameter of a droplet depends on the nozzle hole size "d" for a given frequency of the vibration of the liquid substance and the inlet pressure. In this prior art device where a frequency of around 243 kHz is used, the mean droplet diameter has been found to be around 5 µm, the diameter of the hole of nozzle 9 is around 7 µm and the inlet pressure is a few millibar. One such a droplet thus contains a quantity of around 67 femtolitres (10⁻¹⁵ l) so that as such the number of nozzles may be determined as a function of the amount to be ejected.

Indeed, the fabrication tolerance Δd of the outlet nozzles is an essential factor in controlling and determining the amount, i.e. the volume "V" of an expelled droplet. In fact, this volume V depends on d³ (V= 1/6 * Πd³), d being the diameter of the outlet nozzle. For example, if d = 5 µm, and Δd = ±0.5 µm, the droplet volume V may vary from 47.5 (d= 4.5) to 87 (d=5.5) which is a variation of 83%.

Furthermore, it is known that the pressure drop across the output channel depends on d⁴, so it may be understood that the outlet diameter, the channel diameter, its cross-section, as well as any combination of varying micro-machined cross-sections of the outlet channel and nozzle are an important factor in the structure of the liquid droplet spray device. In fact, this influence of the channel diameter size can be used to determine and vary the pressure drop and the velocity of the nebulized spray.

It is also known that the droplet diameter varies with certain physico-chemical properties of the liquid such as surface tension and viscosity. It is therefore important as shown in the cited prior art to be able to adapt the physical and electrical device parameters (frequency and amplitude) according to the liquid to be expelled and the desired droplet characteristics.

The applicant has now found that under certain circumstances the droplet size, the velocity and flow rate also vary with the voltage applied to the vibrating element. These conditions arise when the pressure drop across the outlet channel is low enough to allow for, for a given range, for example a mean diameter between 5 and 10 µm, the modulation of the droplet size, velocity and flow rate by the energy input alone, hence by the supply voltage.

Consequently, it seems possible to vary the geometric parameters of the outlet means in order to reach a certain droplet diameter, and then vary that size of the ejected droplets by varying the applied voltage.

It is however necessary to apply an innovative micro-machining technique to create a device that reaches such behaviour. Whilst semiconductor fabrication methods such as disclosed by applicant in documents EP-A-0 923 957 and EP-A-1 005 916, cited above, allow to etch the desired straight channels, it is difficult to further improve on the control of the pressure drop while at the same time retaining the control over the droplet size.

It is, therefore, an object of the present invention to provide a method of manufacturing a liquid droplet spray device as well as the thus obtained device which overcomes the above-mentioned inconveniences and which allows for a controllable and programmable droplet size and flow rate.

It is another object of the present invention to provide such a device which is simple, reliable to manufacture, small in size and low in energy consumption and cost.

Thus, the present invention concerns a method of manufacturing a liquid droplet spray device and also a liquid droplet spray device as defined in the appended claims.

Thanks to the specific shape and arrangement of the outlet means of the spray device according to the present invention a programmable droplet size, velocity and flow rate of the expelled droplet may be obtained in a relatively simple manner. This can provide for an economic advantage, as the same device platform can be used to provide a different droplet size distribution, flow rate or exit velocity by simply changing the supply voltage applied to the piezoelectric vibrating element.

Other features and advantages of the liquid spray device according to the present invention will become clear from reading the following description, which is given solely by way of a non-limitative example thereby referring to the attached drawings in which:
- FIGURE 1 is a schematic cross-section of a prior art liquid droplet spray device,
- FIGURE 2a to 2b show schematic detailed views of the top substrate of the prior art liquid droplet spray device of Figure 1,
- FIGURE 3a to 3c show schematic cross-sections of a preferred embodiment of the liquid droplet spray device according to the present invention, and
- FIGURE 4 shows the steps of a method of fabricating, via an inventive differential plasma etch, a possible embodiment of a liquid droplet spray device according to the present invention.
- FIGURE 5 shows a schematic cross-section of a further preferred embodiment of the liquid spray device incorporating a compression chamber and passive valves into the first substrate by applying a further differential step to the same inventive method of fabrication.
- FIGURE 6 shows a bottom view of the first substrate incorporating a compression chamber and passive valves in a single space.

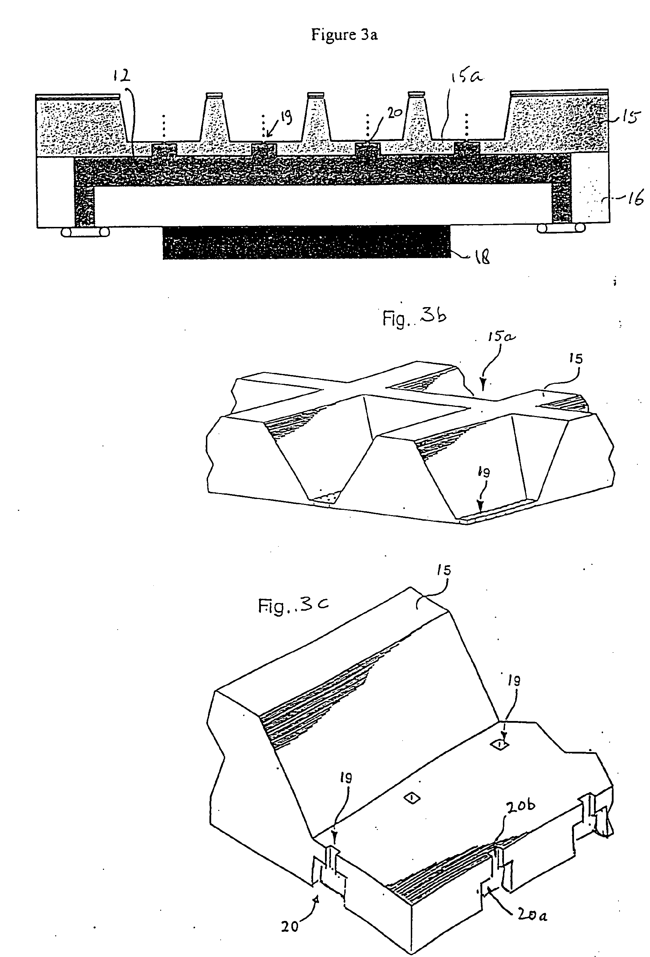

An example of a preferred embodiment will be described hereafter. The present invention thus concerns a method of manufacturing a liquid droplet spray device for nebulizing a liquid substance. The present invention also concerns the thus obtained spray device. For ease of understanding, the structure of the liquid droplet spray device itself will first be described while referring to figures 3a to 3c. In principle, the spray device may be rather similar to the above described prior art spray device, except for the outlet means.

Thus, the spray device according to the present invention also comprises a housing or a substrate. This housing may be formed of a first substrate 15 and a second substrate 16, in a rather similar manner as shown in Figure 1. However, as shown in figure 3, first substrate 15 is placed upside down compared to first substrate 5 of figure 1. The housing may, however, only consist of a single substrate as will be explained in detail later on. Substrate 15 is provided with membrane sections 15a which are thinner sections of the substrate obtained by etching away parts of the substrate using appropriate methods of etch stop to guarantee homogeneous membrane thickness. The manner of obtaining such membrane sections is similar to that as described in the above referenced prior art document EP-A-0 923 957, and is well known to the skilled person from the field of semiconductor etching.

The etching may be done by wet or dry etching resulting in inclined or in straight sidewalls of the substrate portion leading away from the membrane section.

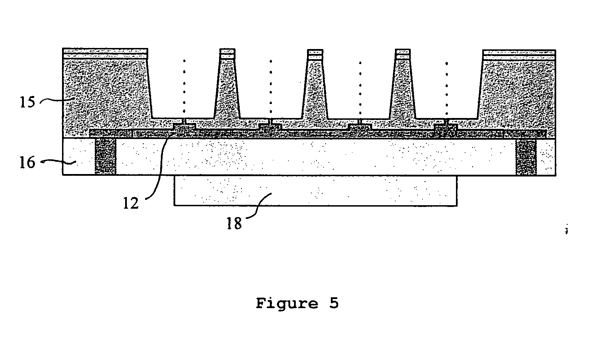

As can be seen in Figure 3, a space 12 for containing the liquid substance is provided within the housing, between the two substrates 15 and 16. Such space can be created by etching away a part of the top surface of second substrate 16 so that when the flat bottom surface of first substrate 15 is attached to the top surface of second substrate 16, this space will be enclosed. In another, preferred embodiment this space is etched totally into first substrate 15, the bottom substrate 16 presenting a flat surface towards the inside of space 12 such as shown in FIGURE 5. The second substrate can also be advantageously replaced directly by the vibrating element 18 which for the purpose of protection has been suitably passivated as described in EP-A-0 923 957.

Space 12 is preferably of a round cross-section in order to facilitate filling and air evacuation and may incorporate a geometry for creating passive valves at its inlet and outlet. It may further be dimensioned or complemented, e.g. by connecting to an internal or external dosage buffer volume 12a, to contain a required dosage amount such that principal volume 12 together with buffer volume 12a correspond to a desired unit dose which may be larger than the internal volume of space 12. Thus, the desired unit dose may have a maximum dosage corresponding to the total volume of space 12, i.e. of principal volume 12 plus buffer volume 12a. In such a way space 12 is at first, e.g. before starting the droplet size generation, completely filled with the liquid to be expelled and remains filled for most of the operation when liquid is expelled roughly as fast as it is aspirated from buffer volume 12a, resulting in a stable operating condition for most of the operation, especially if the content of buffer volume 12a is larger than that of space 12. This buffer volume 12a allows for the spray device to be filled with a different dose for each intended use, ranging from the approximate volume of space 12 to the total volume of space 12 plus buffer volume 12a. Buffer volume 12a is realised preferably as a capillary meander or other geometrical configuration having a cross-section that facilitates capillary action thus providing easier priming of space 12 and buffer 12a. Further, appropriate means, such as a capillary channel 12b for supplying the liquid substance to and allowing exiting from space 12 is provided as known from the mentioned prior art. Such capillary channel can be advantageously configured to act as a passive valve, which is known as such, to allow for the liquid substance to enter and exit the space.

These features are highly advantageous when delivering for example insulin doses with a varying dosage volume depending on the glucometer reading at the time of use. It may of course also be advantageous in other applications where a variable dosage is indicated.

At least one channel outlet nozzle 19 and at least one output channel 20 for connecting space 12 to each outlet nozzle 19 are further provided in the thinner membrane section 15a of substrate 15. It is of course important that the output channel 20 has straight side-walls so as to be able to define the pressure drop across the channel when a droplet is ejected, as already explained in detail in the above mentioned prior art EP-A-0 923 957.

A vibrating element such as a piezoelectric element 18 is disposed on the housing to vibrate the liquid substance in space 12. Preferably, the vibrating element is arranged directly on the first substrate 15 or on a thinner section of the second substrate 16, e.g. acting as a membrane for transmitting a certain compression as well as the vibration to the liquid contained in space 12 and vibration to the total structure such as described in the above-mentioned prior art. When the liquid is excited at an appropriate frequency and under the appropriate pressure, it will be ejected as a spray of droplets through the outlet nozzles with a very low exit velocity. The preferred operation is at the fundamental resonance frequency of vibrating element 18.

According to the present invention, the transition of output channel 20 to outlet nozzle 19 is not only straight, but is also step-shaped. As can be seen in figure 3c, output channel 20 consists of a lower part 20a and an upper part 20b. Lower part 20a of output channel 20 has a larger diameter than upper part 20b and can have the same or a different length as the upper part. Lower part 20a is arranged adjacent space 12 containing the liquid substance which is to be expelled.

Thanks to the stepped shape of the output channel 20, the excited liquid is forced at a higher pressure into the upper part 20b of the output channel. Thus, the eventual size of the droplet results mainly from the liquid volume that is contained in the smaller upper part 20b.

As explained above, for a given liquid the diameter and the volume of the expelled droplet depend mainly on the pressure drop across the output channel and also on the applied voltage, amplitude and frequency.

Thus, as of a certain level of energy balance, the Applicant has found it to be possible, and practical, with the present structure to vary the droplet size by only varying the voltage applied to the vibrating element 18.

Further, thanks to the stepped shape of the output channel, when the same power is used to create the droplet spray, a smaller droplet can be generated as compared to the mentioned prior art devices.

Experiments have shown that, for a given outlet channel diameter, if the applied voltage increases, the mean droplet size decreases. Indeed, the following results were obtained:

| Applied voltage (V) | Mean droplet size (µm) |
|---|---|
| 5 | >18 |
| 10 | 10 - 12 |
| 20 | 7 |
| 30 | 3-4 |

Applicant has found that at virtually no input pressure, at 30 V and approximately 250 kHz applied to the vibrating element, more than 80% of the droplets were smaller than the diameter of the upper part of channel 20b.

It may thus be understood that it is possible to vary the droplet size, but with it the flow rate and also the exit velocity by varying the applied voltage so that a programmable platform for a liquid droplet spray device may be obtained. Consequently, it is possible to provide largely identical liquid droplet spray devices for different applications by simply varying the applied voltage. For example, the same spray device may be used as an inhaler for systemic, deep lung applications which require droplets of less than 3,3 µm or for upper lung treatment which requires droplets of less than 5,6 µm or for various types of liquids which require a different energy input to obtain the same droplet size.

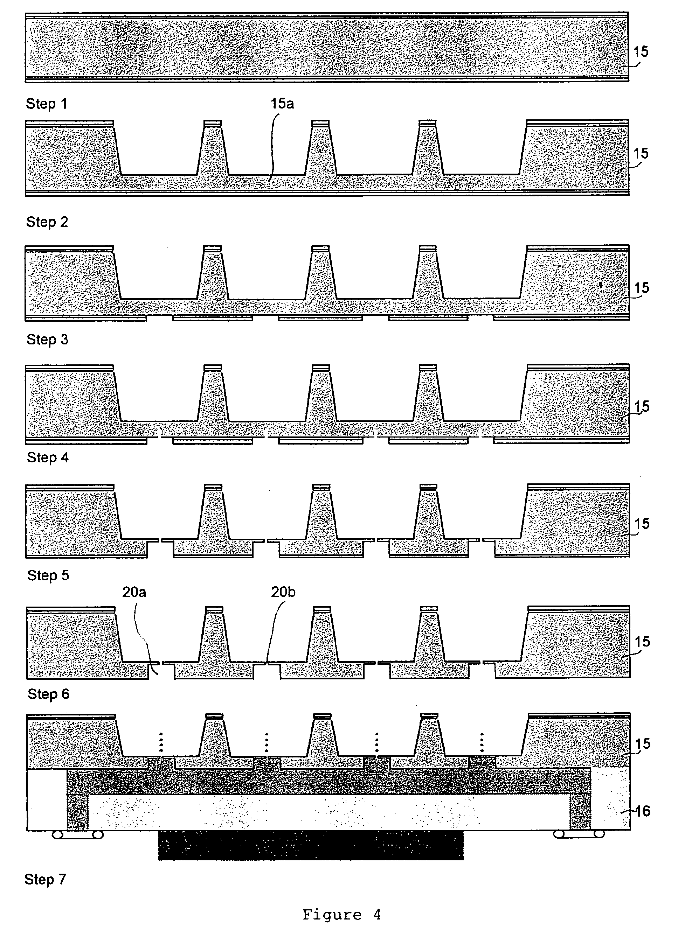

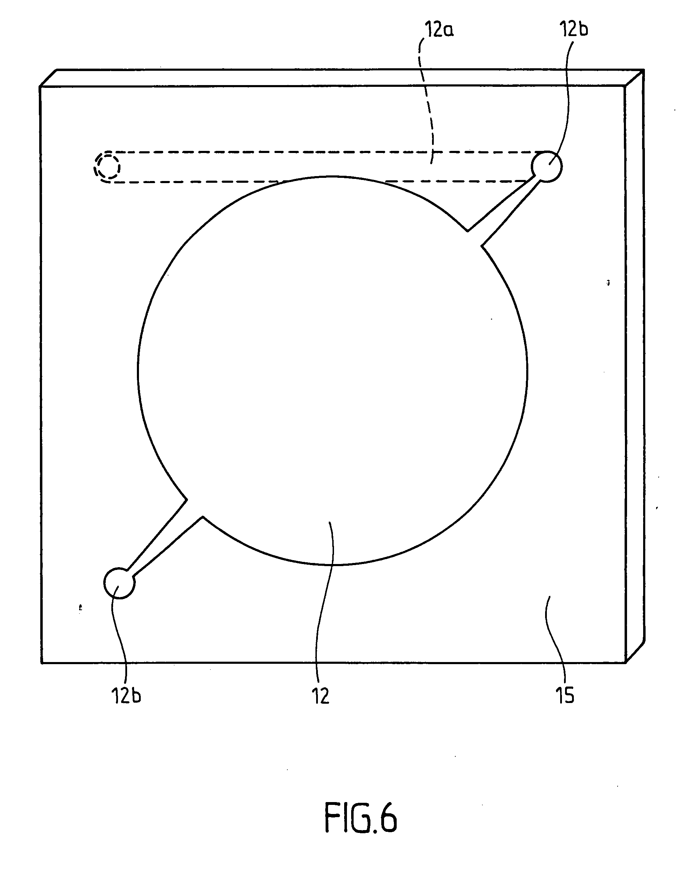

Furthermore, according to the present invention, such a liquid droplet spray device is manufactured by using the following method which is explained whilst referring to FIGURES 4, 5 and 6. In principle, it is possible to etch two silicon wafers, one corresponding to a plurality of first substrates 15 and the other corresponding to a plurality of bottom substrates 16. A detailed explanation of the manufacturing of the first substrate 15 will be provided here. Indeed, this substrate has a more complicated shape than the bottom substrate.

As shown in step 1 of FIGURE 4, first an oxide mask is deposited on both surfaces of a substrate which will form the first substrate 15.

Next, in step 2, the top surface is etched using for example a wet etching process, advantageously with an etch stopper (not shown) so as to obtain the thinner membrane sections 15a.

In step 3, the oxide at the bottom surface of first substrate 15 is opened, defining large openings corresponding to the lower parts 20b of output channels 20. Following this, the oxide mask is etched away to open access to the silicon of substrate 15.

In step 4, after a photo-resist has been applied defining the small openings corresponding to upper parts 20b of output channels 20, a plasma etching, preferably by using an ICP (Inductively controlled plasma) technique, of the silicon is carried out to obtain the shape of the small openings. After stripping of the resist followed by a differential plasma etching, the stepped configuration of output channels 20 with the desired proportions of the parts 20a and 20b is obtained in step 5.

After that last differential plasma etch cycle the wafer is turned and the top surface of the first substrate 15 is etched with the same plasma etcher, preferably in only one step which is stopped in the passivation mode in order to provide a hydrophobic quality of that top surface.

This innovative etching process is called differential etching because the larger exposed surface will be etched faster than the smaller exposed surface, i.e. the "bottom" of the narrow channel part. During this etching step, the narrower channel will be etched to become slightly longer and larger, so as to eventually correspond to the actual desired dimensions of upper part 20b of output channel 20, but this narrower part will also be "pushed forward" with respect to the larger opened portion of the silicon by the etching.

The configuration according to FIGURE 5 is obtained in a similar manner by applying additional steps to the inventive differential plasma process to micro-machine space 12, which acts as a compression chamber, and the passive valves into first substrate 15.

It may thus be understood that by correctly controlling the process the stepped output channels 20 as shown in FIGURE 4 and additionally space 12 which forms the internal compression chamber as shown in FIGURE 5, may be obtained.

As mentioned above, the method of manufacturing of second substrate 16 is simpler. In this respect, the top surface of second substrate 16 may be etched away to create space 12 required for accommodating the liquid substance. A skilled person will readily realise that the thinner section for accommodating the piezoelectric element may be obtained by machining it into silicon or Pyrex wafers. This second substrate may also be directly replaced by a suitably passivated vibrating element 18. Any further shape changes may also be conceived by applying the usual techniques in the field.

Finally, in a further step, indicated as step 7 in figure 4, the bottom surface of first substrate 15 and the top surface of second substrate 16 are bonded together, preferably by using anodic bonding so as to form the housing of the liquid droplet spray device thereby enclosing space 12.

As mentioned above, instead of etching a second substrate to obtain space 12, it is possible to apply a further differential etching step to first substrate 15 after step 6 to etch away a part of first substrate 15 so as to obtain space 12 directly in first substrate 15. It is further also possible to etch, either at the same time or in a separate step, the buffer volume 12a and/or passive valves 12b. Then, second substrate 16 may be applied to first substrate 15 so as to close space 12. However, as also mentioned briefly above, instead of using the second substrate, it is also possible to directly bond the vibrating element 18, whose surface is preferably suitably treated and protected beforehand, to first substrate 15 so as to enclose space 15.

A selective hydrophilic coating, such as an amorphous material such as SiO2, may further be applied to provide a protective layer around the inside surface of space 12 and/or of the output channels 20 to avoid any contamination of the liquid substance by the material of these surfaces and to improve wettability in certain parts. This hydrophilic coating which may be applied as a selective, patterned coating and is advantageously coupled with a selective, patterned hydrophobic coating in certain areas of space 12 and on the outside of first substrate 15.

Alternatively to applying a plasma etch, using the same production equipment as for the innovative differential plasma etching process described earlier, on the top surface of substrate 15 and stopping it in the passivation mode, a hard amorphous carbon film may be provided on first substrate 15 in order to maintain the protective aspect of its surfaces and at the same time to reduce the internal and external stiction due to capillary forces in space 12, and especially on the outside of substrate 15. This hard amorphous carbon film, e.g. diamond-like carbon (DLC) or fluorinated DLC (F*DLC) is provided, preferably in a selective patterned manner in these areas. Other hydrophobic coatings such as nitride or Teflon might be deposited by spinning and curing, by plasma or by other suitable methods. Such selective film coating also allows for a more complete emptying of space 12 and avoids stiction of liquid on the outside surface of substrate 15 due to low surface energy.

The present Applicant has found that such surface property specific coating influences and improves droplet size dispersion and provides an even better monodispersive pattern released by the spray device. These coatings may be carried out as described in the document US-A-5 462 839.

Having described a preferred embodiment of this invention, it will now be apparent to one of skill in the art that other embodiments incorporating its concept may be used. It is felt, therefore, that this invention should not be limited to the disclosed embodiment, but rather should be limited only by the scope of the appended claims.

For example, the same liquid droplet spray device may not only be used for an inhaler in respiratory therapies, but it may generally be used for creating nebulized liquids of different physico-chemical compositions, e.g. using aqueous or alcoholic or other liquid substances.

## Claims

1. Liquid droplet spray device for atomising a liquid substance, comprising:
- a housing consisting at least of a first substrate (15) having at least one membrane section (15a) that is thinner than the rest of said first substrate (15)
- a space (12) within the housing for containing the liquid substance,
- means for supplying said liquid substance to said space (12),
- an outlet means arranged in said membrane section (15a) and comprising at least one outlet nozzle (19) and at least one output channel (20) connecting said space (12) to each of said at least one outlet nozzle (19), said output channel (20) having straight side-walls, and
- a vibrating element (18) disposed to vibrate liquid in said space (12) so as to eject said liquid substance as a spray through said outlet nozzles (19),
**characterised in that** each output channel (20) has a stepped shape having a lower portion (20a) and an upper portion (20b), said lower portion being arranged adjacent said space (12) and being of larger diameter than said upper portion (20b).

2. Liquid droplet spray device according to claim 1, further **characterised in that** said vibrating element (18) is round and of a smaller cross-section than said space (12) and is arranged to operate at its fundamental resonance frequency so as to eject said liquid substance as a spray through said outlet nozzles (19),

3. Liquid droplet spray device according to claim 1 or 2, wherein said housing further comprises a second substrate (16), and wherein said space is arranged between said first and said second substrates (15, 16).

4. Liquid droplet spray device according to claim 1, 2 or 3, further comprising a passive valve (12b) arranged in physical combination with said space (12) for facilitating the homogeneous operation of said device by providing a homogenous filling of said space (12) with said liquid substance.

5. Liquid droplet spray device according to anyone of the preceding claims, wherein said space consists of a principal volume (12) and a buffer volume (12a), said buffer volume being dimensioned such that the total volume of said principal volume (12) together with said buffer volume (12a) corresponds to a maximum dosage, so that a desired variable dosage depending on the specific usage of said spray device may be contained in said space, said variable dosage being equal to or less than said maximum dosage.

6. Method of manufacturing a liquid droplet spray device for atomising a liquid substance according to claim 1,: the method comprising the following steps:
a. depositing an oxide mask on both surfaces of a silicon substrate which will form the first substrate (15),
b. etching the top surface of said first substrate (15) so as to obtain the thinner membrane sections (15a),
c. applying a first photo-resist to the bottom surface of said first substrate (15) defining large openings corresponding to the lower parts (20b) of said output channels (20) and then etching away the oxide mask to open access to the silicon of said first substrate (15),
d. applying a second photo-resist to define small openings corresponding to the upper parts (20b) of said output channels (20) and then using a partial plasma etching of the silicon to obtain a narrow channel which is narrower and shorter than the actual upper part (20b) will be, and stripping the second photo-resist,
e. etching the opened silicon by using a differential plasma etching,
f. providing means for supplying said liquid substance to said space (12), and
g. providing said vibrating element (18).
h. bonding together said first substrate (15) and said vibrating element (18) to form said housing of said liquid droplet spray device.

7. Method of manufacturing a liquid droplet spray device for atomising a liquid substance according to claim 1,: the method comprising the following steps:
a. depositing an oxide mask on both surfaces of a silicon substrate which will form the first substrate (15),
b. etching the top surface of said first substrate (15) so as to obtain the thinner membrane sections (15a),
c. applying a first photo-resist to the bottom surface of said first substrate (15) defining large openings corresponding to the lower parts (20b) of said output channels (20) and then etching away the oxide mask to open access to the silicon of said first substrate (15),
d. applying a second photo-resist to define small openings corresponding to the upper parts (20b) of said output channels (20) and then using a partial plasma etching of the silicon to obtain a narrow channel which is narrower and shorter than the actual upper part (20b) will be, and stripping the second photo-resist,
e. etching the opened silicon by using a differential plasma etching,
f. etching a second silicon substrate so as to form said second substrate (16),
g. bonding together said first substrate (15) and said second substrate (16) to form said housing of said liquid droplet spray device,
h. providing means for supplying said liquid substance to said space (12), and
i. providing said vibrating element (18).

8. Method of manufacturing a liquid droplet spray device according to claim 6 or 7, further comprising, after step "e" and before step "g", a step of applying a selective hydrophilic coating to provide a protective layer around the inside surface of space 12 and/or of the output channels 20 to avoid any contamination of the liquid substance by the material of these surfaces and to improve wettability in certain parts.

9. Method of manufacturing a liquid droplet spray device according to claim 8, further comprising the step of applying a selective hydrophobic coating in those areas of said space (12) which do not contain the hydrophilic coating and on the outer surface of said first substrate (15).

10. Method of manufacturing a liquid droplet spray device according to claim 8, further comprising an additional step of plasma etching the top surface of said first substrate (15) which is stopped in the passivation mode to provide a hydrophobic surface quality using the same production equipment as for the other differential plasma etch steps.

11. Method of manufacturing a liquid droplet spray device according to claim 9 or 10, further comprising the step of applying a hard amorphous carbon film on said hydrophobic coating.
